# EUROPEAN PATENT APPLICATION

(11) **EP 1 229 115 A1**
(43) Date of publication of application: **07.08.2002**
(21) Application number: 00963023.7
(22) Date of filing: 02.10.2000
(51) Int. Cl.: C12N 15/12, C07K 14/47, C12Q 1/68, A61K 38/17, A61P 31/00, A61P 37/04, A61P 43/00

(54) **NOVEL C-TYPE LECTIN AND GENE THEREOF**

(30) Priority: 15.10.1999 JP 29372499
(71) Applicant: Japan Science and Technology Corporation, Kawaguchi-shi, Saitama 332-0012 (JP)
(72) Inventor: AKIRA, Shizuo, Minoo-shi, Osaka 562-0031 (JP); MATSUMOTO, Makoto, Nishinomiya-shi, Hyogo 663-8126 (JP)
(74) Representative: Cresswell, Thomas Anthony
(86) International application number: JP0006820
(87) International publication number: WO0127271

(57) **Abstract**

A novel C-type lectin "Mincle" which is formed from a transcriptional target gene of a nuclear factor interleukin 6 (NF-IL6) in a peritoneal macrophage (PMΦ); a gene encoding the same; and medicinal compositions containing the same. A protein which is capable of activating macrophage and induced by a nuclear factor NF-IL6; gene DNA encoding the above protein or polynucleotides consisting of at least 14 bases of the above DNA; medicinal compositions containing the above-described protein and pharmaceutically acceptable carriers; and an antibody against the above protein. This novel protein "Mincle" is useful as a regulator in an immune system, in particular, an immune system under the transcriptional regulation by the nuclear factor NF-IL6.

## Description

### Technical Field

The present invention relates to a novel protein which is a target substance downstream of a nuclear factor NF-IL-6 and is one of C-type lectins, a gene encoding the same and medicinal compositions containing the same.

### Background Art

Interactions of proteins with sugars have many functions in an immune system. Many of animal lectins (sugar binding proteins) regulate intercellular interactions utilizing structural relations of regions for pathogenic recognition and regions for Ca²⁺-dependent sugar recognition (C-type, CRDs)(Weis, W. I., et al., Immunol. Rev., 163:19-341, 1998).

Macrophages express various C-type lectins such as macrophage mannose receptor, macrophage asialoglycoprotein binding protein (M-ASGP-BP) and the like. Macrophage mannose receptor is involved in primary defense by direct phagocytosis against pathogens (Tayler, M., Carbohydrate-recognition proteins of macrophages and related cells. Blood cell biochemistry [Horton, M., Ed.] 5 vols, Plenum Press, New York).

Mannose receptor which binds to sugars such as mannose, N-acetylglucosamine, fucose, and the like do not form a basic moiety as an oligosaccharide in mammals, but is often found on the surfaces of microorganisms.

N-ASGP-BP is expressed on the surface of activated macrophages, recognizes a terminal galactose/N-acetylgalactosamine unit, and is involved in an interaction of tumoricidal macrophages with tumor cells (Oda, s., et al., J. Biochem [Tokyo] 104:600-5, 1988; Li, M., et al., J. Biol. Chem., 265(19):11295-8, 1990; Sato, M., et al., J. Biochem [Tokyo] 111(3):331-6, 1992).

On the other hand, NF-IL-6 (nuclear factor-interleukin 6) is primarily found as a nuclear factor bound to interleukin 1 (IL-1) responsive element (around -150 bp, ACATTGCACAATCT) located in the promoter of interleukin 6 (IL-6) gene (Ishii, H., et al., Mol. Cell. Biol., 10(6):2757-64, 1990). NF-IL-6 was cloned, and human NF-IL-6 is composed of 345 amino acids, has a basic amino acid rich region involved in DNA binding at the C terminus, and side by side the leucine zipper structure (bZIP domain) which is a protein-protein interaction domain exists. And it has been shown that its sequence is homologous to CCAAT/enhancer binding protein (C/EBP)and that it is a member of a basic leucine zipper family of the transcription factors (Akira, S., et al., EMBO J., 9(6):1897-1906, 1990). NF-IL-6 was also reported as AGP/EBP, LAP, IL-6DBP, rNFIL-6, C/EBPβ, and CRP2 by the other groups (Chang, C. J., et al., Mol. Cell. Biol., 10(12):6642-53, 1990; Descombes, P., et al., Genes Dev., 4(9):1541-51, 1990; Poli, V. et al., Cell, 63(3):643-53, 1990; Metz, R., et al., Genes Dev., 5(10):1754-66, 1991; Gao, Z., et al., Genes Dev., 5(9):1538-52, 1991; Williams, S. C., et al., Genes Dev., 5(9):1553-67, 1991).

C/EBP, NF-IL/6β, Ig/EBP and CHOP in addition to NF-IL6 have been reported as genes which exhibit high homology to the leucine zipper structure, and they form a NF-IL6 family (also referred to as C/EBP family).

NF-IL6 motif is also observed in the promoter regions of IL-1, TNF, IL-8, G-CSF in addition of IL-6, and IL-6 responsive element of various acute proteins as well as lysozyme induced along with macrophage activation and the enhancer region of NOS gene, and thus, NF-IL6 appears to play important roles in inflammation and immune responses.

NF-IL6 has been known to be phosphorylated and activated by MAP kinase cascade, however, NF-IL6 has been also shown to be phosphorylated by PKC, PKA and the like in addition to MAP kinase cascade. This suggests that NF-IL6 is the target of several different signal transductions.

However, what gene expression is controlled by signalling to NF-IL6 in vivo is not yet demonstrated in detail. It has been reported that one of the targets of NF-IL6 transcription is presumed to be leukocyte colony stimulating factor (G-CSF) in macrophages (Tanaka. T., Akira, S., et al., Cell, 80(2):353-61, 1995), but no detail has not been known yet.

The studies using NF-IL6 knockout mice (cited above, Cell 80(2):353-61, 1995) are noticed in respect to this issue. NF-IL6 knockout mice are normally born even at less Mendelian ratio by heterologous crossings.

NF-IL6 knockout mice (NF-IL6 deficient (-/-) mice) have been found to be highly sensitive to air infection of pathogens such as Listeria monocytogenes, Candida albicans and the like, and it was demonstrated that this is attributed to lack of intracellular bactericidal ability of macrophages.

Peritoneal macrophages (PMΦ) in NF-IL6 deficient (-/-) mice appear to lack intercellular bactericidal ability against Listeria monocytogenes and to lose tumor killing activity and tumor static activity. However, the macrophages used in this study produced nitrogen oxides at a normal quantity which has an important role to kill intercellular bacteria and parasites, and this suggests that there is another pathway depending on NF-IL6 but not nitrogen oxides to exert Listeria bactericidal ability and tumor killing activity.

Overexpression of NF-IL6 protein demonstrated that IL-6, macrophage chemotactic protein (MCP-1), macrophage inflammatory protein-1α (MIP-1α), MIP-1β, osteopontin, CD14, and lysozyme are the genes downstream of NF-IL6 in some hematopoietic cells. However, on the contrary, the expression levels of these genes in macrophages from NF-IL6 deficient (-/-) mice are sometimes comparable to those in macrophages from the wild type (WT) mice. This is believed because the other C/EBP family gene such as C/EBPδ partially compensates NF-IL6 deficiency (Hu, H. M., J. Immunol., 160(5):2334-42, 1998).

Thus, NF-IL6 plays an important role to activate macrophages, and important are not only elucidating the immune system in inflammation and infection but also elucidating target substances of NF-IL6 as substances to control and regulate the immune system.

As the result of intensive study to elucidate a target gene of NF-IL6 transcription and demonstrate its translation product, the present inventors have found a novel glycoprotein which is one of C-type lectins and named Mincle (macrophage inducible C-type lectin).

### Disclosure of the Invention

The present invention provides a novel C-type lectin "Mincle" which is produced from a transcriptional target gene of nuclear factor interleukin 6 (NF-IL6)in peritoneal macrophages (PMΦ); a gene encoding the same; and medicinal compositions containing the same. The novel protein "Mincle" of the present invention is useful as a regulator in an immune system, in particular the immune system under the transcriptional regulation by the nuclear factor, NF-IL6.

The present invention relates a protein having an amino acid sequence represented by the sequence No.2 in the sequence table or an amino acid sequence wherein one or two or more amino acids are added, substituted or deleted in the above amino acid sequence, having an ability for macrophage activation and inducible by NF-IL6.

The present invention also relates a gene DNA encoding the above-described protein of the present invention, preferably DNA having a base sequence represented by the sequence No.1 in the sequence table, or DNA capable of hybridizing to the genes under a stringent condition, or these genes, preferably polynucleotides consisting of at least 14 bases of the DNA.

Further, the present invention relates medicinal compositions containing the above-described protein of the present invention and pharmacologically acceptable carriers; and an antibody against the above protein.

### Brief Description of the Drawings

Fig. 1 is photos as an alternative to drawings which show expression of Mincle mRNA of the present invention in PMΦ at various time periods after the treatment with LPS.
Fig. 2 is photos as an alternative to drawings which show expression of Mincle mRNA in the wild type and NF-IL6 deficient (-/-) type PMΦ treated with various inflammation induction factors.
Fig. 3 is photos as an alternative to drawings which show the result of Northern blot analysis for Mincle expression in various cell lines.
Fig. 4 denotes the base sequence of 2.5-kb murine Mincle (m-Mincle) cDNA. The first base denotes a transcription initiation site, and is determined by a primer-extension method described below. The underlined part in Fig. 4 denotes polyadenylation sites, and the capital under the bases indicates an encoded amino acid by the single character code. The asterisk denotes the termination codon.
Fig. 5 denotes the comparison of the amino acid sequence of human Mincle with that of murine Mincle of the present invention. The black parts indicate that the sequences are homologous between human and mouse Mincles, and the gray parts indicate that amino acids are conserved in both. The underlined part indicates a transmembrane region, the arrow indicates an initiation site of C-type lectin, a black triangle indicates a homologous region in an N-glycosylation site between human and mouse Mincles, an open triangle indicates a glycosylation site only in human, asterisks indicate deduced phosphorylation sites by protein kinase C.
Fig. 6 shows the amino acid sequences comparatively displaying the carboxy terminus of m-Mincle protein (m-Mincle), the CRC region of mMCL as well as three group-II C type lectins, murine CD23 (mCD23), murine asialoglycoprotein receptor 1 (mASGP-1) and murine macrophage asialoglycoprotein binding protein (mM-ASGP-BP).
Fig. 7 shows the map of Mincle of the present invention in the end region of murine chromosome 6. The upper panel of Fig. 7 shows the segregation of gene pairings of Mincle and franking genes in 95 backcrossed animals typed for all locations. The lower panel of Fig. 7 shows a part of the genetic map of chromosome 6 indicating the locations of Mincle and genes linked it.
Fig. 8 shows the result of flow cytometric analysis studying surface expression of murine Mincle Flag protein of the present invention.
Fig. 9 is a photo as alternative to a drawing which shows the result of detection of expression of m-Mincle Flag protein of the present invention by the Western blot analysis.
Fig. 10 is a photo as alternative to a drawing which shows the result of the primer-extension analysis using a primer corresponding to the nucleotides from 81 to 112 of the cDNA to determine the transcription initiation site of m-Mincle gene of the present invention.
Fig. 11 shows the sequence in the 5' franking region of m-Mincle gene of the present invention. The arrow in Fig. 11 denotes the transcription initiation site. Open square denotes TATA box, and methionine (Met) is displayed under the translation initiation codon (ATG). The sites capable of binding various transcription factors are represented by the underlines.
Fig. 12 shows the result of the test of transcription induction of the murine Mincle promoter by NF-IL6 in a trans activation assay.
Fig. 13 is photos as an alternative to a drawing which shows the result of the test for NF-IL6 binding to the positions from -66 to -58 of the m-Mincle promoter.
Fig. 14 is photos as an alternative to a drawing which shows the result of the test conducted using purified GST-NF-IL6 fusion protein to confirm that NF-IL6 protein binds to P1 oligonucleotide.

### Best Mode for Carrying Out the Invention

NF-IL6 plays an important role to activate macrophages, and important are not only elucidating the immune system in inflammation and infection but also elucidating target substances of NF-IL6 as substances to control and regulate the immune system. However, no target substance of NF-IL6 could be found.

The present inventors have found a novel protein (hereinafter referred to as "Mincle"), one of C-type lectins which is inducible by NF-IL6, and a gene encoding the same by the subtraction cloning method using NF-IL6 deficient (-/-) mice, and studied their characteristics.

First, the present inventors carried out the subtraction cloning to compare expression differences of mRNA in the wild type and NF-IL6 deficient (-/-) type PMΦ. PMΦ from the wild type and NF-IL6 deficient (-/-) type mice were stimulated with 100 ng/ml of LPS and 100 U/ml of IFN-γ for 16 hours. Tester cDNA and driver cDNA are synthesized using poly (A)⁺ RNA extracted from the wild type and NF-IL6 deficient (-/-) type PMΦ, respectively. Subtraction hybridization was carried out by concentrating cDNA existing dominantly in the tester cDNA. In order to decrease errors due to individual differences (false positive), two subtraction libraries were used. Differential hybridization for 1,000 colonies was carried out in each library, and positive clones were sequenced. The clones separated from both libraries were noticed among the positive clones. Five cDNA were found of which expression was dramatically reduced in NF-IL6 deficient (-/-) type PMΦ compared to the wild type PMΦ. One of those cDNA is novel, and was named as "macrophage inducible C-type lectin (Mincle)" based on characteristics of its expression and the primary structure of the protein encoded by it.

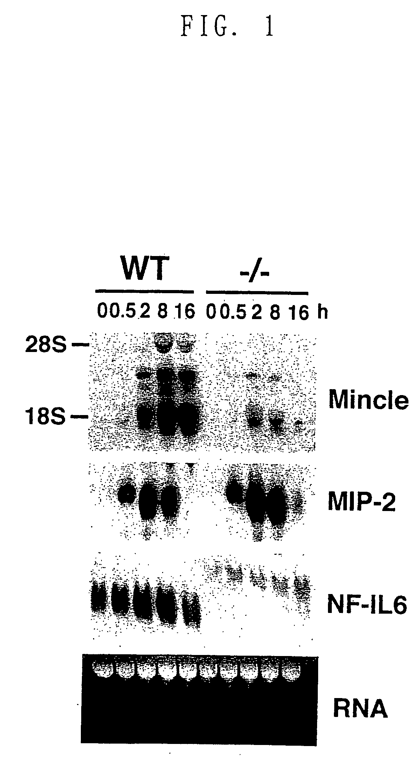

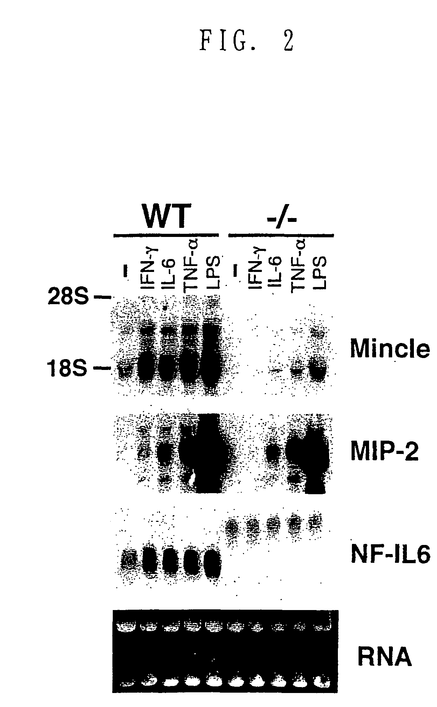

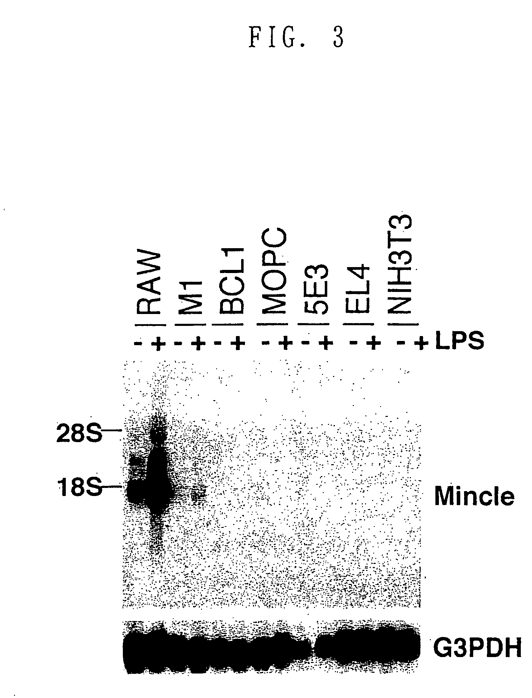

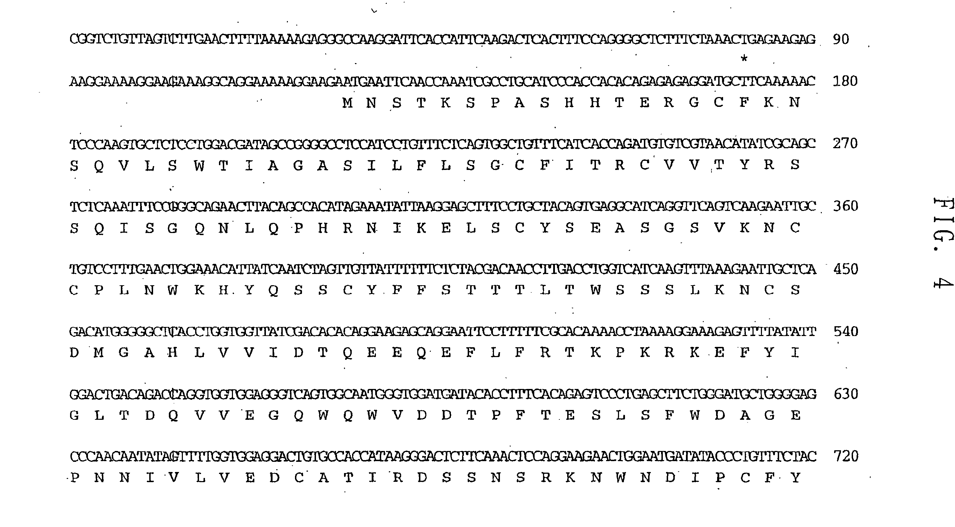

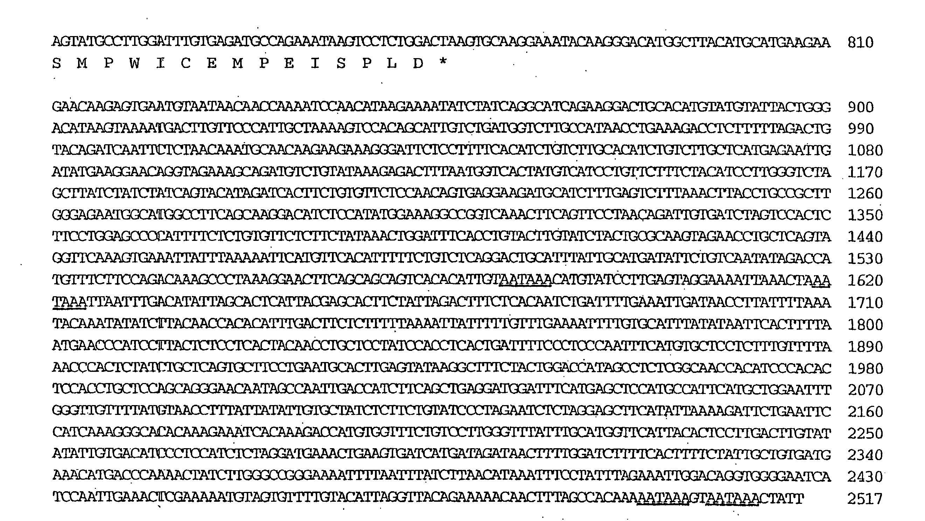

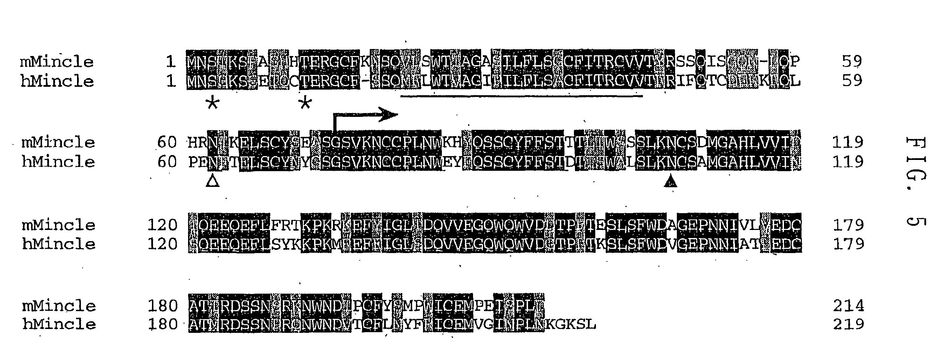

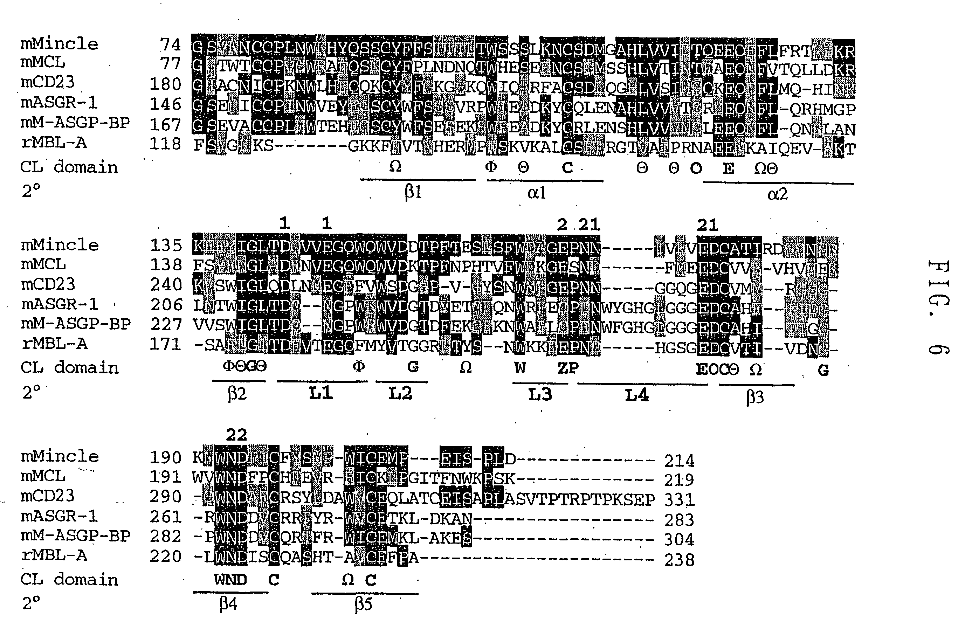

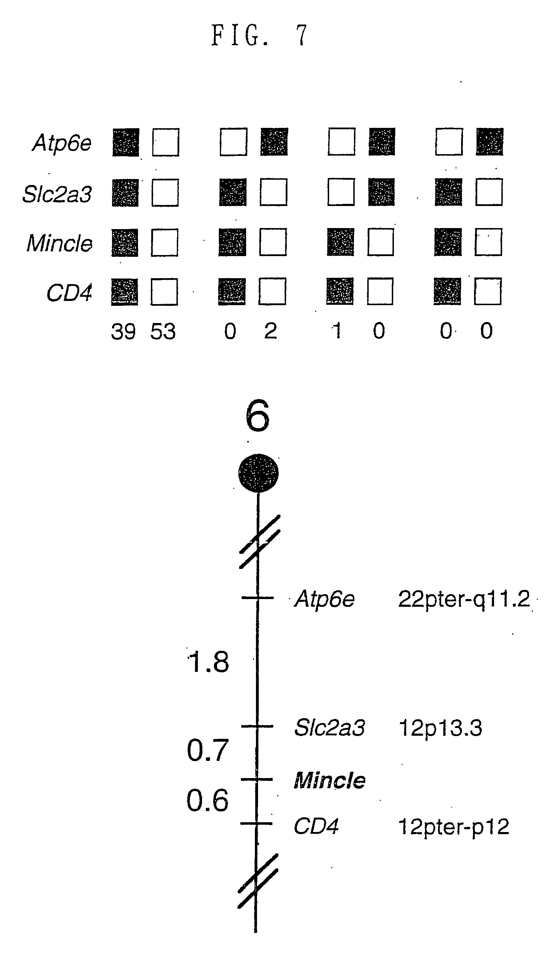

The expression of Mincle was evaluated by Northern blotting of PMΦ stimulated with LPS. Although the expression of Mincle mRNA is difficult to be detected in PMΦ unstimulated with LPS, it can be detected at 2 hours after the treatment with LPS. This result is shown in Fig. 1. Fig. 1 shows the expression of Mincle mRNA in PMΦ at various time periods after the treatment with LPS. That is, the wild type and NF-IL6 deficient (-/-) type PMΦ were treated with 100 ng/ml of LPS for an indicated time, respectively, total RNA was extracted, electrophoresed (5µg/lane), transferred to a nylon membrane and hybridized with a Mincle specific probe. MIP-2 was used as a positive control to confirm that PMΦ used effectively responded to LPS. The staining with ethidium bromide at the lower panel in Fig. 1 is for confirming that the quantities of samples were equal.

Three types of mRNA were hybridized with this probe. Among them, the shortest mRNA (1.7 kb) was most frequently observed. The expression of Mincle mRNA in the wild type PMΦ could be retained for at least 16 hours after the stimulation with LPS. The levels of NF-IL6 mRNA was increased within 30 min after the treatment with LPS before introducing Mincle mRNA into the wild type PMΦ (see Fig. 1). In NF-IL6 deficient (-/-) type PMΦ, the expression of Mincle mRNA was induced at 2 hours after the stimulatory treatment with LPS, but its levels were considerably lower than those in the wild type. It was shown that NF-IL6 deficient (-/-) cells effectively responded to the stimulation with LPS by comparing the quantities of MIP-2 mRNA in both type cells.

Next, the expression of Mincle mRNA in the wild type and NF-IL6 deficient (-/-) type PMΦ was examined after the treatment with various inflammatory factors. After the cells were stimulated with IFN-γ, IL-6, TNF-α and LPS for 4 hours, respectively, total RNA was extracted, and Northern blotting was carried out using a Mincle specific probe.

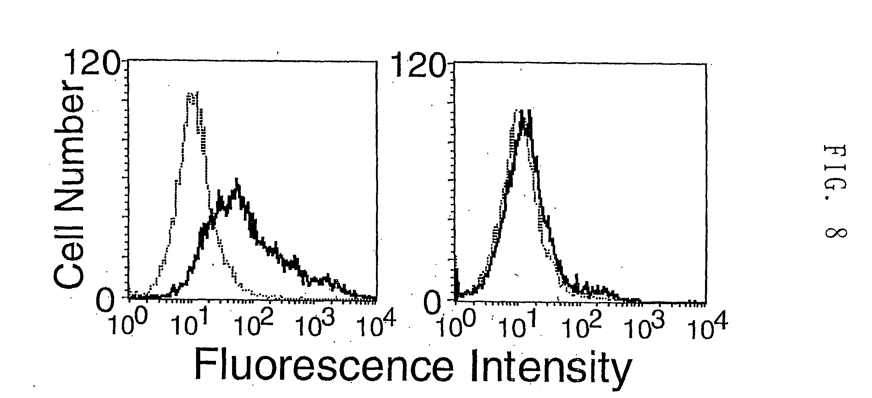

This result is shown in Fig. 2. Fig. 2 shows the expression of Mincle mRNA in the wild type and NF-IL6 deficient (-/-) type PMΦ treated with various inflammatory factors. That is, the cells were stimulated with medium alone (-), IFN-γ (250 U/ml), IL-6 (2000 U/ml), TFN-α (5000 U/ml) and LPS (100 ng/ml) for 4 hours, respectively, total RNA was extracted, this was electrophoresed, transferred to the nylon membrane and hybridized with the Mincle specific probe. MIP-2 was used as a positive control to confirm that PMΦ used effectively responded to LPS. The staining with ethidium bromide at the lower panel in Fig. 2 is for confirming that the quantities of samples were equal.

As the result, strong induction of Mincle mRNA was observed in the wild type PMΦ treated with IFN-γ, IL-6, TNF-α or LPS. On the contrary, only extremely low levels of mRNA were detected in NF-IL6 deficient (-/-) type PMΦ. The treatment with IL-1β, phorbol myristate acetate or ionomycin could not significantly induce Mincle mRNA in both the wild type and NF-IL6 deficient (-/-) type PMΦ.

These results indicate that some inflammatory factors strongly induce Mincle mRNA expression depending on NF-IL6. Since the treatment with LPS induced the expression of Mincle most strongly in PMΦ, the expression of Mincle after the treatment with LPS was tested using various cell lines.

The result is shown in Fig. 3. Fig. 3 shows the result of Northern blotting for Mincle expression in various cell lines. The cases of the treatment (+)of RAW264.7 (transformed peritoneal macrophage), M1 (myeloblastic leukemia), BCL1 (mature B cell), MOPC (myeloma [melanoma]), 5E3 (natural killer cell), EL4 (thymoma), and NIN3T3 (fibroblast) with LPS (100 ng/ml) for 4 hours and the non-treated cases (-) are shown. After the treatment, total RNA was electrophoresed (5 µg/lane), transferred to the nylon membrane and hybridized with the Mincle specific probe. The lower panel in Fig. 3 shows the control hybridized with G3PDH. The quantity of G3PDH was fewer in 5E3 cells compared to the other cells.

The expression of Mincle was observed in macrophages, RAW264.7 cells and its level was rapidly increased after the stimulation with LPS. Low levels of the expression were also detected in M1 myeloblastic leukemia cells. However, no detectable level of Mincle mRNA was expressed in the other cell lines, BCL1 (mature B cell), MOPC (myeloma [melanoma]), 5E3 (natural killer cell), EL4 (thymoma), and NIH3T3 (fibroblast). Moreover, Mincle mRNA was not detected in brain, heart, lung, spleen, kidney, skeletal muscle, and testis. This indicates that the expression of Mincle mRNA might be limited to macrophages.

The present inventors cloned 2.5- and 1.7-kb murine Mincle (m-Mincle) cDNA by RACE method. As the result by analyzing these sequences, it was demonstrated that the difference in both is attributed to alternate sequences of polyadenylation site in 3'-non-translation region but not to splicing.

The base sequence of 2.5-kb murine Mincle (m-Mincle) cDNA is shown in the sequence No.1 in the sequence table. The first base indicates the transcription initiation site, and this was determined by the primer-extension method described below. The underlined parts in Fig. 4 denote the polyadenylation sites, and the capital under the bases represents an encoded amino acid by the single character code. The asterisk denotes the termination codon. The shorter 1.7-kb one is the sequence of the base number from 1 to 1641. This sequence was deposited in DDBJ/GenBank/EMBL database as the accession No. AB024717.

Both 2.5- and 1.7-kb cDNA have the open reading frame of 642 bp encoding the polypeptide consisting of 241 amino acids, and the molecular weight of the polypeptide was estimated as about 24.4 kDa.

The amino acid sequence of this polypeptide is shown as the sequence No.2 in the sequence table.

This polypeptide does not possess hydrophobic signal peptides at the NH₂-terminus whereas it has the transmembrane region consisting of 24 amino acids predicted by the sequence of the hydrophobic amino acids. From the primary structure of Mincle, Mincle was found to be the type II membrane-binding protein having the intracellular region consisting of 21 amino acids at the NH₂-terminus and the extracellular region consisting of 169 amino acids at the COOH-terminus.

The present inventors identified its human homologue by combining degenerative PCR and RACE methods using cDNA from THP-1 cells stimulated with LPS. The identified human Mincle (h-Mincle) consists of 219 amino acids, and its amino acid sequence is shown in Fig. 5 by displaying with murine sequence. The comparison of the amino acid sequences from human and murine Mincle is shown in Fig. 5. The black parts denote that the sequences are homologous between human and murine Mincles, and the gray parts denote that the amino acids are conserved in both human and mouse. The underlined part denotes the transmembrane region, the initiation site for C-type lectin region is denoted by an arrow. The same N-glycosylation site in human and murine Mincle is indicated by a black triangle, and the N-glycosylation site only in human is indicated by an open triangle. The predicted sites as phosphorylation sites by protein kinase C are indicated by asterisks. The nucleotide sequence of human Mincle was deposited to DDBJ/GenBank/EMBL database as accession No. AB024718.

The present inventors cloned 2.2- and 1.0-kb human Mincle (h-Mincle) cDNA. Both had the identical open reading frame of 657 bp encoding the polypeptide consisting of 219 amino acids. Comparing the amino acid sequences between h-Mincle and m-Mincle, 67% of the sequences were identical and 85% were homologous (see Fig. 5).

m-Mincle appears to have an N-glycosylation site at Asp 107 whereas h-Mincle appears to have the sites at Asp 62 and Asp 107. The two potential phosphorylation sites by protein kinase C (Ser 3 and Thr 12) exist in the intracellular region of murine and human Mincle (see Fig. 5). These sites were compatible with the phosphorylation motif of protein kinase C (Ser/Thy-X-Arg/Lys).

As the result from conducting homology search by available databases, no sequence identical to the amino acid sequences of Mincle of the Presert invention was found. However, the sequence in the extracellular region of Mincle was found to be homologous to those in various C-type lectins. Murine and human Mincle have the CRDs consisting of 136 and 141 amino acids, respectively. And this region has extremely high homology to macrophage C-type lectin (MCL) which is related to group-II C-type lectins (Balch, S. G., et al., J. Biol. Chem., 273(29):18656-64, 1998).

In Fig. 6, the amino acid sequences are shown by displaying the carboxy terminus of m-Mincle protein of the present invention, the CRD moiety of mMCL, as well as three group-II C-type lectins, murine CD23 (mCD23), murine asialoglycoprotein receptor (mASGR-1) and murine macrophage asialoglycoprotein binding protein (mM-ASGP-BP). The sequence of rat mannose binding lectin A (rMBL-A) of which detail analysis was reported is also displayed in Fig. 6 (Weis, W. I., et al., Science, 254(5038):1608-15, 1991; Iobst, S. T., et al., J. Biol. Chem., 269(22):15505-11, 1994; Iobst, S. T., et al., J. Biol. Chem., 269(22):15512-9, 1994; Weis, W. I., et al., Structure, 2(12):1227-40, 1994).

Fig. 6 shows the comparison of the amino acid sequences of m-Mincle of the present invention with Ca²⁺-dependent sugar recognition sites of C-type lectins (C-type, CRDs) known in the art, and each sequence is displayed from the position corresponding to the initiation amino acid of C-type lectin region of m-Mincle. The black parts indicate that the sequences are identical to that of m-Mincle, and the gray parts indicate that the sequences are similar to that of m-Mincle. "CL domain" indicates symbols used for the C-type lectin region. Amino acids are displayed by the single character code (Z denotes E or Q). The conserved regions are represented by Greek characters or O (Φ; Θ; Ω; and O denote aromatic series; aliphatic series; aromatic or aliphatic series; and containing oxygen, respectively). The secondary structure (2°) and calcium binding region indicate those determined by Weis using rMBL-A (Weis, W. I., et al., Science, 254(5038):1608-15, 1991). The calcium binding sites (1 and 2) to the side chains contributing to an oxygen ligand are displayed on these sequences.

The determined Mincle sequence was 44%, 40%, 38% 36% and 31% identical with that of CRD of mMCL, mCD23, mASGR-1, mM-ASGP-BP, and rMBL-A, respectively. For m-Mincle protein, 12 sites of 14 sites of C-type lectins shown here are identical except the open triangle at the 153 and Gln at the 189 positions, and 18 parts of highly conserved 18 part amino acid sequences were conserved (see Fig. 6) (Drickamer, K., Prog. Nucleic Acid Res. Mol. Biol., 45:207-32, 1993). m-Mincle protein is associated with rMBL-A and CRD to conserve all amino acid sequence for retaining calcium cation. From these results, Mincle of the present invention can be considered as one type of C-type lectins.

The genomic DNA fragment can be obtained by the promoter analysis. This genomic DNA includes first five exon regions (nucleotides form 1 to 611) of m-Mincle in addition to the 5'-flanking region. The first three exon regions correspond to the regions having each function of receptor polypeptides (cytoplasmic end, membrane intra and extra linkage and extracellular region), respectively. This is also characteristic for group-II C-type lectins (Bezouska, et al., J. Biol. Chem., 266(18):11604-9, 1991). The positions for the forth and fifth exon regions are completely icentical to those observed in CRD of chicken hepatic lectin which is an original form of group-II C-type lectins.

Next, the chromosomal location of Mincle gene was confirmed.

The chromosomal location of murine Mincle gene was determined by interspecies backcrossing using progenies of cross between species[(C57BL/6J x Mus spretus)F1 X C57BL/6J].

Fig. 7 shows a mapping of Mincle gene at the end region of murine chromosome 6. The upper panel of the Figure indicates the segregation of gene pairings of Mincle and its flanking genes in 95 backcrossed animals performed their typing for all locations. Each column indicates a chromosome identified in a progeny from the backcrossing inherited from F1 (C57BL/6J x Mus spretus) parents.

Black squares indicate the presence of an allele from C57BL/6J, and open squares indicate the presence of an allele from Mus spretus. The number of progenies having the shown type of chromosome is expressed under each column. A part of genetic map of the chromosome 6 indicating the locations of Mincle and genes linked with Mincle is shown in the lower panel of Fig. 7. The numerical numbers at the left side indicate the distance of genetic recombination between the locations of genes as centimorgan unit, and those at the right side indicate the locations in human chromosome.

Mapping of interspecies backcrossing produced the types of 2700 positions or more. These were distributed on all X chromosome as well as autosomal chromosomes (Copeland N. G., and Jenkins N. A., Trends Genet., 7(4):113-8, 1991). In order to obtain information for RFLP (restriction fragment length polymorphism), analysis was carried out by the Southern blot hybridization using the probe of murine Mincle cDNA and digested DNA from C57BL/6J and Mus spretus with various enzymes. RFLP of 6.5 kb BamHI fragments from Mus spretus (see Example) was used for analyzing the position of Mincle in hybrid mice. As the result from the mapping, it was shown that Mincle locates on the end region of the murine chromosome 6 where Atp6e, Slc2a3 and Cd4 are linked. Ninety-five mice were analyzed for each marker and provided for segregation analysis (see Fig. 7), but typing of up to 167 mice was carried out as pairs of these markers. Each location was analyzed as a combination for pairings using more data for recombination frequencies. The ratio of total mice exhibiting recombinant chromosome to all mice analyzed for each pair in the location and the most probable gene sequence is centromere-Atp6e-2/110-Slc2a3-1/136-Mincle-1/167-Cd4. The recombination frequencies [represented the gene distance as centimorgan (cM) ± standard deviation] are Atp6e- 1.8±1.3- Slc2a3- 0.7±0.7- Mincle-0.6±0.6- Cd4.

The map of the chromosome 6 in the animals obtained in the experiment was compared with the murine genetic map made from the reports of mapping location in many animals which were not cloned (provided from the mouse database which is the computerized database in Jackson Laboratory in Bar Harbor, ME). Mincle was mapped in the composite map region in the mouse with no variant of the phenotype anticipated by variants in this locus. The end region of the murine chromosome 6 has a regional homology to the human chromosomes 22 and 12 (see Fig. 7). This suggests that human Mincle might be mapped on two chromosomes.

The surface property of m-Mincle was studied.

The amino acid sequence of Mincle suggests that Mincle is a type II membrane protein as described above. In order to confirm the surface property of Mincle protein, m-Mincle cDNA tagged with carboxy terminal Flag is transfected into 293 T cells. And Mincle-Flag protein was detected by flow cytometric analysis using anti-Flag M2 antibody without filtrated cells.

Fig. 8 shows the result of flow cytometric analysis in which the surface expression of murine Mincle-Flag protein was examined. Transformed 293 cells with m-Mincle-Flag (left side) and 293 cells mock-transfected (right side) were incubated with biotin-conjugated anti-Flag M2 antibody (solid line) or control buffer alone. The expression intensities were compared after labeling with FITC-streptoavidin.

The cells transfected with m-Mincle-Flag are stained with M2 antibody, indicating that m-Mincle-Flag protein has strong surface expression. The cells mock-transfected are not stained with M2 antibody (Fig. 8 right panel).

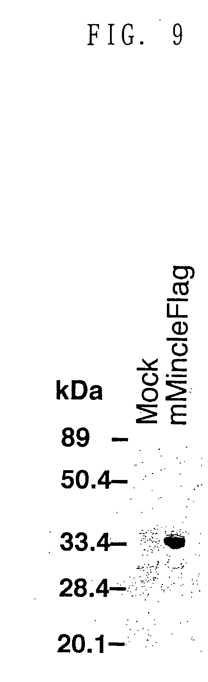

At the same moment, the expression of m-Mincle-Flag protein was detected by Western blotting analysis. The result is shown in Fig. 9. Cell lysates of 293 cells transfected with m-Mincle-Flag or mock-transfected were immunologically precipitated with anti-Flag M2 antibody, electrophoresed on SDS-polyacrylamide gel, transferred to a nitrocellulose membrane, and m-Mincle-Flag protein was detected with biotin-conjugated anti-M2 antibody, and further incubated with horseradish peroxidase-conjugated streptoavidin. The immunoreactivity was visualized by chemiluminescence.

The observed molecular weight of m-Mincle-Flag protein corresponds to about 35 kDa. However, estimated molecular weight of m-Mincle-Flag is 25.4 kDa. This result suggests that m-Mincle protein might be considerably modified after translation.

Next, the transcription initiation site of Mincle gene was determined.

Primer-extension analysis was carried out using a primer corresponding to cDNA nucleotide from 81 to 112 to determine the transcription initiation site of m-Mincle gene.

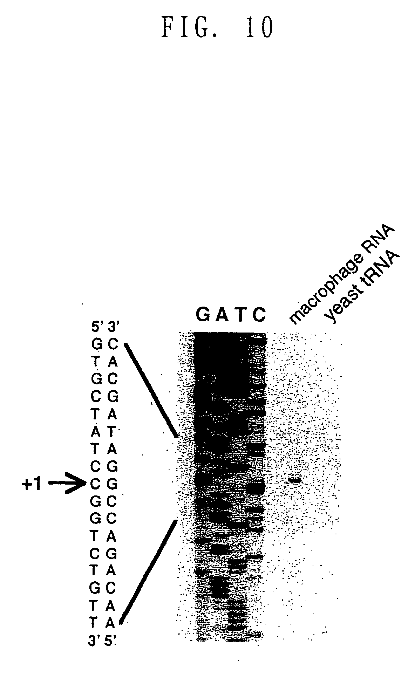

Total RNA obtained from PMΦ stimulated with LPS was hybridized with ³²P-labeled antisense oligonucleotide complementary to cDNA nucleotide from 112 to 81, and subsequently extended the primer with a reverse transcriptase. The product was electrophoresed along with the dideoxy-sequencing ladder produced from the same oligonucleotide. An arrow indicates the transcription initiation site. The product from the negative control reaction (yeast t-RNA replacing PMΦ RNA) did not exhibit a band. The result is shown in Fig. 10.

As a negative control, the same reaction was carried out using yeast t-RNA. The product obtained from the extension reaction was shown by displaying with m-Mincle antisense sequence produced from the same oligonucleotide primer (see Fig. 10). As the result, one transcription initiation site was determined, and it was the site corresponding to 125th base upstream of the translation initiation codon. A nucleotide at this site is C (cytosine) and the transcription initiation site was tentatively named as +1.

The sequence analysis of the 5' flanking region was carried out.

Murine genomic DNA library was screened by ³²P-labeled EcpRI-EcoRI DNA fragment (nucleotide from 126 to 496) of m-Mincle. Sequencing of the isolated clone contains the flanking region and first five exon regions. The 1.8 kb promoter region of m-Mincle gene was completely sequenced from the both strands.

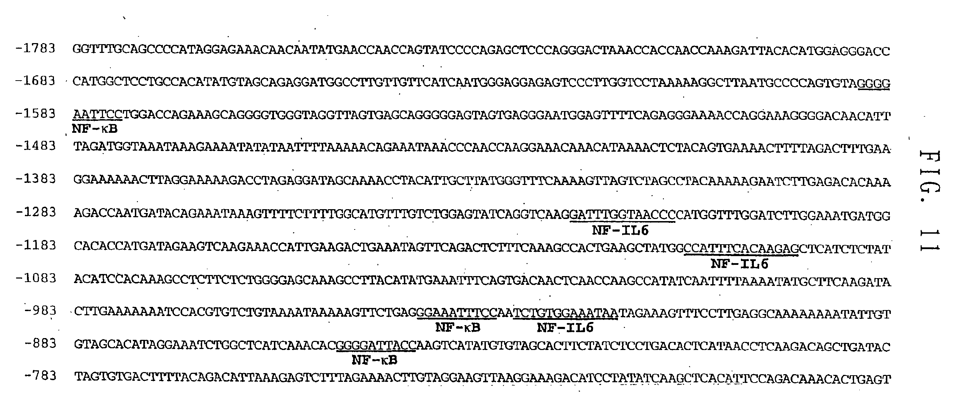

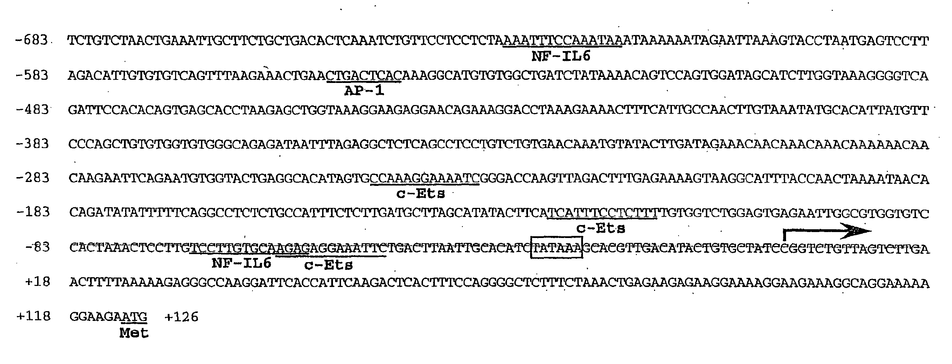

The determined sequence is shown in Fig. 11. An arrow in Fig. 11 shows the transcription initiation site. An open square indicates TATA box, and methionine (Met) was displayed under the translation initiation codon (ATG). The sites to where various transcription factors can bind were underlined. This sequence was deposited to DDBJ/GenBank/EMBL as the accession No. AB024719.

It was found that there are potential binding sites for various transcription factors by computer search using TFSEARCH program (Heinemeyer, T., et al., Nucleic Acids Res., 26(1):362-7, 1998). Some locations are demonstrated which are involved in expression regulation of this gene with LPS. The motifs included in them are NF-IL6, nuclear factor-kappa B (NF-κB), activator protein-1 (AP-1) and the site to where c-Ets binds. The deduced binding motif of NF-IL6 appears to be located at from -1222 to -1210, from -1108 to -1095, from -929 to -917 and from -632 to -619. The standard TATA box is located at from 29 to 24 bp upstream of the transcription initiation site.

Next, the expression induction of NF-IL6 which activates the m-Mincle promoter was studied.

The m-Mincle promoter-luciferase plasmids deleting various 5' parts were constructed to examine whether NF-IL6 trans-activates the m-Mincle promoter or not. The 1852 bp fragment containing 1783 bp of 5' flanking region and 69 bp of 5' non-coding region was fused to luciferase without promoter, reporter or vector, pGL3, and luciferase expression systems partially deleting various 5' parts were prepared.

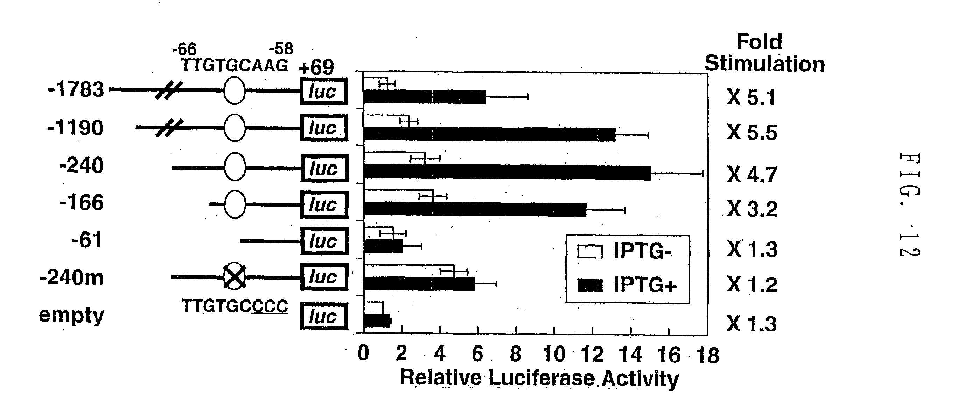

Fig. 12 shows the results in which transcription induction of the murine Mincle promoter by NF-IL6 was tested in the trans-activation assay. The left side of Fig. 12 indicates m-Mincle promoter-luciferase constructs, and from the upper, they denote sequentially pGL3-1783/+69, -1190/+69, -240/+69, -166/+69, -61/+69; NF-IL6 binding site variant construct, pGL3-240m; and pGL3 vector. The number indicates the relative location from the transcription initiation site. Open ovoid circles indicate NF-IL6 binding site (from -66 to -58). Renilla luciferase vector (pRL-SV40) (20 µg) as a control of transfection and 20 µg of the construct was co-transfected in NFIL6 M1 cells which express NF-IL6 by the IPTG treatment using an electroporation method. After the transfection, cells were divided into two aliquots, and cloned in the presence or absence of IPTG (1 mM). After 18 hours, the cells were collected and extract solutions were prepared. The levels of luciferase activity were standardized by activity values of co-transfected Renilla luciferase, and shown as the values correlated with those of pGL3 basic vector without promoter. The resultant data are shown as mean ± SD from three time experiments. The values of fold stimulation shown in the right side in Fig. 12 are those calculated by dividing an activity quantity in the presence of IPTG with that in the absence of IPTG as a ratio corresponding to the expression of NF-IL6 in each construct.

NF-IL6 M1 cells have been known to induce and express human NF-IL6 protein within 4 hours after the addition of IPTG (Matsumoto, M., Sakano, et al., Int. Immunol., 10(12):1825-35,1998).

The transfectants of 9GL3-1783/+69, -1190/+69, -240/-69, and -66/+69 showed from about 3 to 5 fold induction by NF-IL6 expression. The deletion from -166 to -61 positions in the sequence completely reduced a reactivity by IPTG (see Fig. 12). This region includes one NF-IL6 binding motif (TKNNGNAAK) from -66 to -58 positions. NF-IL6 consensus sequence was introduced into pGL3 -240/+69 vector to prepare a variant pGL3 -240m. The residues consisting of two adenine residues and one guanine residue, which are believed to be NF-iL6 binding site were all replaced with cytosine residues. An activity of pGL3 -240m was assayed and it was found not to be involved in NF-IL6 expression (see Fig. 12). As the result, it was shown that NF-IL6 binding site from -66 to -58 positions is required for the promoter activity of m-Mincle.

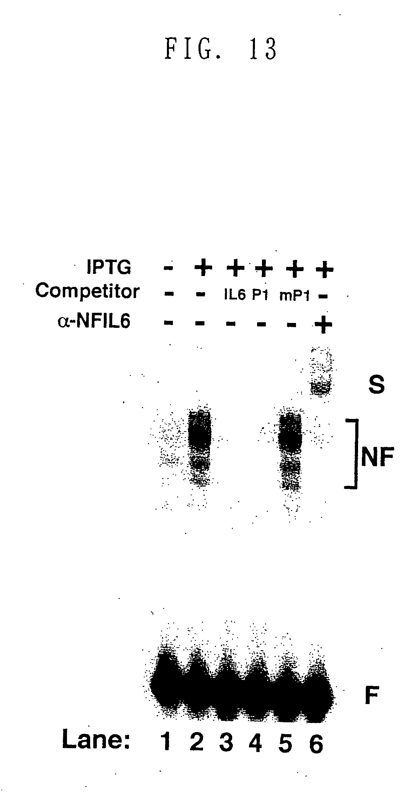

Furthermore, NF-IL6 binding to the m-Mincle promoter from -66 to -58 positions was studied. It was tested whether NF-IL6 reacts to the m-Mincle promoter from -66 to -58 positions or not. The result is shown in Fig. 13.

That is, the double strand oligonucleotide P1 corresponding to the sequence from -76 to -45 positions was radioactively labeled with ³²P, and incubated with nuclear protein prepared from NFIL6 M1 cells as a control (lane 1) or NFIL6 M1 cells treated with IPTG for 12 hours (lanes 2 to 6). The double strand oligonucleotide from human IL-6 promoter (IL-6), P1 or mutated P1 (mP1) was added as a competitor at 100 fold molar excess to P1 probe (lanes 3 to 5). In lane 6, a rabbit polyclonal antibody against NF-IL6 was added to the binding reaction. The locations for super shifted complex (S), probe-nuclear protein complex (NF) and free probe (F) were described on the right side in Fig. 13, respectively.

As shown in Fig. 13, a broad binding activity appears when P1 probe is incubated with the nuclear extract prepared from NF-IL6 M1 cells treated with IPTG (lane 2 in Fig. 13). Congenital NF-IL6 binding from human IL-6 promoter (IL-6), P1 and mutated P1 (mP1 with the same mutation as is the case with the reporter gene assay was carried out for comparison. Unlabeled IL-6 and P1 oligonucleotides at 100 fold molar excess were competed with the nuclear protein-DNA complex (lanes 3 and 4 in Fig. 13). However, mP1 could not eliminate the nuclear protein-DNA band (lane 5 in Fig. 13). The rabbit polyclonal antibody against NF-IL6 inhibits the generation of these complex, but forms the other greatly shifted complex (lane 6 in Fig. 13).

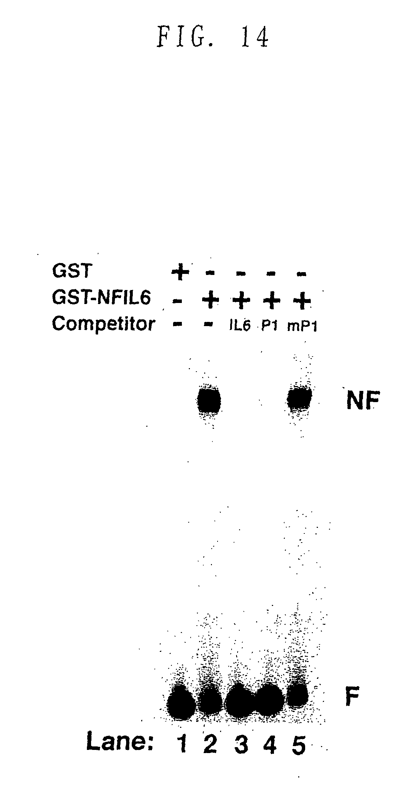

In order to further confirm that NF-IL6 binds to P1 oligonucleotide, the test was carried out using purified GST-NF-IL6 fusion protein instead of the nuclear extract solution of NF-IL6 M1 cells. The result is shown in Fig. 14.

That is, P1 probe was incubated with GST protein (lane 1) or GST-NF-IL6 fusion protein (lanes 2 to 5). Unlabeled IL-6, P1 and mP1 at 100 fold molar excess were added to this binding reaction (lanes 3 to 5). The locations of the probe-GST-NF-IL6 fusion protein complex (NF) and free probe (F) were showed on the right side in Fig. 14.

As the result, it was found that NF-IL6 protein formed a specific complex with the DNA probe (lane 2 in Fig. 14). Unlabeled IL6 or P1 oligonucleotide can eliminate the DNA-protein complex by competition, but mP1 oligonucleotide can not (lanes 3 to 5 in Fig. 14). These results demonstrate that NF-IL6 binds to the -66 to -58 positions of the m-Mincle promoter, and that is consistent with the report that NF-IL6 binds to the consensus sequence.

One objective of the present invention is to define the functions of NF-IL6 in macrophages through identifying the target downstream of NF-IL6. The novel C-type lectin, i.e., Mincle of the present invention was isolated by the subtraction cloning method. And this is extremely reduced in its expression responding to the inflammatory stimulus in NF-IL6(-/-) macrophages. The expression of Mincle gene is strongly induced by bacterial lipopolysaccharide, and some inflammatory cytokines including IFN-γ, IL-6, TNF-α in the wild type PMΦ. In the present invention, the expression of Mincle mRNA was observed only in PMΦ treated with the inflammatory product, macrophage cell line, RAW264.7, and myeloblastic leukemia cell line. M1. The expression of Mincle gene may be limited to cells belonging to myeloid monocytes stimulated with an inflammatory mediator.

Macrophages infiltrate into various inflammatory areas in which concentrations of inflammatory cytokines are high, such as rheumatoid arthritis (Chu, C. Q., et al., Clin. Exp. Immunol., 86(3):380-6, 1991), various tumors (Mantovani, A., et al., Immunol. Today 13(7):265-70, 1992), wounds (Leibovich, S. J., et al., Prog. Clin. Biol. Res., 266:131-45, 1988). Mincle normalizes these states, and plays some roles in macrophage infiltration. NF-IL6 deficient (-/-) macrophages lack bactericidal activity and cytotoxicity for tumors even if they are treated with LPS or IFN-γ to be significantly activated. Low levels of Mincle induction in NF-IL6 deficient (-/-) peritoneal macrophages (PMΦ) indicates a possibility that Mincle might play some roles in bactericidal activity and cytotoxicity for tumors. Anticipated functions of Mincle are to recognize hydrocarbons on the surfaces of microorganisms and tumors and subsequently activate macrophages. Activated macrophages lyse and kill bacteria and tumor cells as if natural killer cells recognize target cells through natural killer receptor protein 1 and kill tumor and viral cells (Chambers, W.H., et al., J. Exp. Med., 169(4):1373-89, 1989; Ryan, J. C., et al., J. Immunol., 147(9):3244-50, 1991).

Mincle is included in such an immune surveillance process by activating macrophages under the transcriptional control of NF-IL6. However, more studies are required for accurately describing biological functions of Mincle.

Although Mincle is the target gene of NF-IL6 in addition to G-CSF, the sequence of Mincle promoter is quite different from that of G-CSF. The G-CSF promoter comprises a segmentary between the -165 and -196 bp positions, the promoter element 1 of G-CSF gene (GPE1), and plays an important role to induce the expression of G-CSF gene by LPS (Nishizawa, M., et al., Mol. Cell Biol., 10(5):2002-11, 1990). The subsequent study of GPE1 demonstrated that both the NF-κ binding element in GPE1 and an element proximal to NF-IL6 are important for induction of the G-CSF promoter by TNF-α and IL-1β (Shannon, M. F. et al., Growth Factors, 7(3):181-93, 1992).

Both NF-κBp65 and NF-IL6 bind to GPE1 to form the complex of DNA with the three. Mincle is presumed to have three sites of NF-κB binding motifs in its 1.7 kb promoter region, and these sites exist separately from the element essential for NF-IL6 binding. There is also a possibility that NF-κB might induce the transcription signal of Mincle gene in inflammation. However, NF-κB does not interact directly with NF-IL6 on the Mincle promoter. The Mincle gene promoter potentially has elements capable of being bound by the other transcription factors such as Ap-1 and c-Ets which activate inflammation. These transcription factors may act to induce Mincle gene in macrophages in cooperation with NF-IL6. In the latest studies, it has been shown that activities of C/EBPα, -β, -δ, and -ε are not required for the expression of IL-6 and MCP-1 (Williams, S. C., et al., J. Biol. Chem., 273(22):13493-501, 1998; Hu, H. M., et al., J. Immunol., 160(5):2334-42, 1998).

Ectopic expression of C/EBPα, -β, -δ, and -ε is enough for the expression of IL-6 and MCP-1 induced by LPS in P388 lymphoblasts. P388 lymphoblasts usually lack C/EBP factors and do not produce inflammatory cytokines induced by LPS. In fact, it has been shown that C/EBPδ exhibits a similar expression pattern as NF-IL6 and is involved in control of some genes induced at inflammation (Kinoshita S., et al., Proc. Natl. Acad. Sci. USA 89(4):1473-6, 1992). This is similar to the fact that deficit of NF-IL6 is partially covered with C/EBPδ induction by the LPS treatment in vivo. No precise mechanism has not been defined yet in which the other NF-IL6 family protein covers the expression of IL-6 and MCP-1 but not the expression of Mincle and G-CSF in macrophages activated with LPS.

The sequence of Mincle protein is highly similar to those of group-II C-type lectins which frequently mediate glycoprotein endocytosis. The prototype of this group is asialoglycoprotein receptors, of which surface proteins of hepatic cells bind to galactose ends, and glycoproteins circulate on exposed receptors.

The receptors are delivered to lysosomes via endocyte by their internalization but not by direct changes of serum glycoproteins. Human asialoglycoprotein receptor H1 subunit contains cytoplasmic tyrosine at the position 5 in the internalization motif for the receptor. (46) It has been generally said that rapid internalization of the cellular surface requires a short stretch of amino acids including tyrosine. The other members of group-II C-type lectins such as M-ASGP-BP (4,4, Li, M., Kurata, H., Itoh, N., Yamashina, I., and Kawasaki, T., J. Biol. Chem., 265(19):11295-8, 1990), CD23(48, 48. Kikutani, H., Inui, S., Sato, R., Barsumian, E. L., Owaki, H., Yamasaki, K., Kaisho, T., Uchibayashi, N., Hardy, R. R., Hirano, T., and et al., Cell, 47(5):657-65, 1986), gp120 C-type binding lectin (49, 49. Curtis, B. M., Schamowske, S., and Watson, A. J., Proc. Natl. Acad. Sci. USA 89(17:8356-60, 1992), Kupffer cell fucose receptor (50, 50. Hoyle, G. W., and Hill, R. L., J. Biol. Chem., 263(16):7487-92, 1988) have tyrosine residue(s) in the cytoplasmic region. However, Mincle have no tyrosine residue in the cytoplasmic region. This indicates that Mincle protein does not undergo sufficiently endocytosis. The other C-type lectin which has no tyrosine in the cytoplasmic region is MCL. This protein linkage exhibits the highest concordance with Mincle.

Mincle and MCL exhibit some general characteristics. For example, their protein sequences show high homology with those of group-II C-type lectins; transcription is highly expressed in macrophage areas; no tyrosine residue is contained in the cytoplasmic region; and that murine genes are mapped on the chromosome 6. Thus, Mincle and MCL can be classified as belonging to group-II C-type lectins. As the results, we have isolated Mincle which is a C-type lectin which responds to an inflammatory product and is strongly induced under the regulation cf NF-IL6 in the macrophage area, and demonstrated its natures. Identification of Mincle ligand and actions to be targets of Mincle gene will help to describe its physiological roles in vivo.

The Mincle protein of the present invention has an activation action for macrophages, and is useful as therapeutic and preventive agents for various immune disorders such as infectious diseases and inflammation, inflammatory diseases. The medicinal composition of the present invention consists of Mincle of the present invention and pharmaceutically acceptable carriers. Various additives used for formulation can be used as pharmaceutically acceptable carriers.

The medicinal compositions of the present invention can be appropriately formulated into injection agents, suppositories, liquid agents and the like depending on their dosing forms. The medicinal composition of the present invention can be administered by various methods, but parenteral administration is preferable.

The dosage of the medicinal composition of the present invention depends on states of patients and types of diseases, but the equivalent dosage as the usual protein preparations can be administered.

The present invention provides a gene having a partial sequence of a gene encoding the protein of the present invention. The gene encoding the protein of the present invention can exemplify, but is not limited to, DNA described as the sequence No.1 in the sequence table. The polynucleotide consisting of the partial sequence of the present invention includes those containing at least 14 bases or more, preferably 20 bases or more, and more preferably 30 bases or more. The preferable part of the partial sequence can be appropriately chosen depending on an intended purpose of the polynucleotide. For example, those consisting of partial complementary chains in the non-translation region are preferable when the partial polynucleotides of the present invention are used as antisense, and the part having a specific sequence of the gene of the present invention is preferable when used as a probe. Further, the partial sequence polynucleotide of the present invention can be also used as a promoter at PCR and the like.

Further, the present invention provides an antibody against the protein of the present invention. As a sensitizing antigen for producing the antibody of the present invention, the full-length of the protein of the present invention may be used, but the partial length of the peptide from the amino acid sequence can be used. Rabbits, rats, mice and the like are immunized with this sensitizing antigen, and subsequently a polyclonal or monoclonal antibody can be obtained by the standard method.

### Examples

Next, the present invention is more specifically described by examples, but the present invention is not limited to these examples.

The reagents used in the following examples were obtained by the methods described as follows.

Lipopolysaccharide (LPS) (E. coli 055: B5) and thioglycolate broth (Brewer's formula) were purchased by Difco (Detroit MI). Interferon-γ (IFN-γ), granulocyte macrophage-colony stimulating factor (GM-CSF), IL-6 and tumor necrosis factor-α (TNF-α) were obtained from Genzyme (Cambridge, MA). Restriction and DNA modification enzymes were products of Toyobo (Otsu, Japan). Oligonucleotides were synthesized by Nisshinbo (Tokyo). Isopropyl-β-D-thiogalactoside (IPTG) was a product of nacalai tesque (Kyoto, Japan). Streptomycin and Penicillin G were from Meiji Seika (Tokyo). [α-³²P]dCTP (3000Ci/mmol) and [γ-³²P]ATP (3000Ci/mmol) were obtained from Amersham Pharmacia Biotech (Little Chalfont, UK).

The NF-IL6 (-/-) mice generated by homologous recombination have been described previously (18, 18. Tanaka, T., Akira, S., Yoshida, K., Umemoto, M., Yoneda, Y., Shirafuji, N., Fujiwara, H., Suematsu, S., Yoshida, N., and Kishimoto, T., Cell 80(2):353-61, 1995). NF-IL6 (-/-) mice and WT mice were littermates derived from intercrossing hemizygous females and males.

### Example 1 (Preparation of PMΦ)

PMΦ were collected by peritoneal lavage with phosphate-buffered saline (PBS) at 4 days after intraperitoneally injecting 2 ml of sterile thioglycolate to 8-12 week-old mice. PMΦ were plated on 10-cm plastic dishes at 2.5 X 10⁶ cells/dish in macrophage culture medium (MCM).

MCM consists of Dulbecco's modified Eagle's medium (DMEM; Nissui. Tokyo) supplemented with 10% fetal bovine serum, 2 mM L-glutamine, 50 U/ml of GM-CSF, 100 µg/ml streptomycin, and 10 U/ml penicillin G. After 2 h incubation to allow for adherence of macrophages, the dishes were washed vigorously to remove nonadherent cells. Fresh MCM was added on day 2, and fresh MCM without GM-CSF was added on day 4 of culture. PMΦ were treated with appropriate reagents on day 5 for harvesting RNA.

### Example 2 (Cell Lines)

Murine B cell leukemia (BCL-1), myeloma cells (MOPC 315), thymoma cells (EL-4) and human monocytic leukemia cells (THP-1) were cultured in RPMI 1640 medium (Gibco/BRL, Gaithersburg, MD) containing 10 % fetal bovine serum, 100 µg/ml streptomycin, and 10 U/ml penicillin G. Murine natural killer cells (5E3) were cultured in RPMI 1640 supplemented with 10 % fetal bovine serum and 500 U/ml of IL-2 (Genzyme). NFIL6M1 cells were cultured in minimum essential medium (Gibco/BRL) supplemented with twice the normal concentration of amino acids and vitamins, 10 % fetal bovine serum, 400 µg/ml of Geneticin (Gibco/BRL) and 50 µg / ml of hygromycin B (Boehringer Mannheim, Mannheim, Germany) (21, 21. Matsumoto, M., Sakao, Y., and Akira, S., Int. Immunol., 10(12):1825-35,1998).

Murine macrophage cells (RAW264. 7), embryonic fibroblast cells (NIH3T3) and human embryonic kidney cells (293T) were cultured in DMEM supplemented with 10 % fetal bovine serum, 100 µg/ml streptomycin, and 10 U/ml penicillin G.

### Example 3 (Construction of Subtracted cDNA Library and Differential Screening)

All procedures were performed essentially according to PCR-Select cDNA subtraction kit (Clontech, Palo Alto, CA).

WT and NF-IL6 (-/-) PMΦ) at about 80% confluence were treated with 100 ng/ml LPS and 100 U/ml IFN-γ for 16 h.

Total RNA was prepared by RNAeasy Kit (Qiagen, Hilden, Germany), following poly (A)⁺ RNA selection using Oligotex™-dT30 Latex beads (TaKaRa, Otsu, Japan). Tester and driver cDNAs were synthesized from 2 µg of poly (A)⁺ RNA of WT PMΦ and NF-IL6 (-/-) PMΦ, respectively.

To select for NF-IL6 inducible transcripts, Rsa I digested tester cDNA was ligated to oligonucleotide linkers and hybridized with excess driver cDNAs.

After hybridization, differential transcripts were selectively amplified using Advantage cDNA Polymerase Mix (Clontech).

The primary polymerase chain reaction (PCR) was done for 27 cycles and the nested PCR for 10 cycles on a thermal cycler (Gene Amp 9600; Perkin-Elmer, Norwalk, CT). Products from the nested PCR were inserted into pT7Blue Tvector (Novagen, Madison, WI).

Independent clones were amplified by direct colony PCR and differential screening against 1000 clones was performed according to manufacture's instruction. Briefly, colony PCR products were dot-blotted onto HybondN+ membranes in duplicate. Each DNA dot blot was hybridized with itself ("+" subtracted probe) or with reverse-subtracted cDNA ("―" subtracted probe).

To make the "―" subtracted probe, subtractive hybridization is performed with the original tester cDNA as a driver and the driver cDNA as a tester. Clones that hybridize only with "+" probe were sequenced by autosequencer utilizing dye-labeled dideoxynucleotides (Applied Biosystems PRISM 377 DNA Sequencer). Sequence comparisons against DDBJ databases were obtained from the DDBJ blast server.

### Example 4 (Rapid Amplification of cDNA Ends (RACE))

Rapid amplification of cDNA ends (5'-RACE and 3'-RACE) were performed using a Marathon cDNA Amplification Kit (Clontech). Using 1 µg of poly(A)⁺ RNA from WT PMΦ stimulated with 100 ng/ml LPS and 100 U/ml IFN-γ, a library of adaptor-ligated double stranded cDNA was constructed as described by the manufacture's instruction.

To obtain a full length cDNA of mouse Mincle (mMincle), an antisense primer, 270F (5'-GAGAAAATGGGGCTCCAGGAAGAGTG-3') and a sense primer, 92R (5'-CCCTAAAGGAACCTTCAGCAGCAGTC-3') were designed from the sequence of the cDNA fragment obtained by subtraction cloning for 5'-RACE and 3'-RACE, respectively.

PCR reaction was performed in a 50 µl reaction mixture containing 40 mM Tricine-KOH, pH 9.2, 15 mM KOAc, 3.5 mM Mg(OAc), 75 µg/ml bovine serum albumin, 200 µM dNTPs, 0.2 µM 270F or 92R, 0.2 µM adaptor prime 1 (Clontech), 5 µl of diluted adaptor-ligated double stranded cDNA solution, 1 µl of 50X Advantage cDNA Polymerase Mix (Clontech), PCR conditions were 94°C 30s, followed by 5 cycles of 94°C 5s and 72°C 2min, followed by 5 cycles of 94°C 5s and 70°C 2min, and followed by 20 cycles of 94°C 5s and 68°C 2min.

The resulting 1312 bp and 1039 bp products were purified from an agarose gel, subcloned into pT7Blue Tvector, and sequenced.

To obtain human Mincle (hMincle), cDNA synthesized from THP-1 cells mRNA
Extracted after LPS-stimulation (5µg/ml) was subjected to PCR using degenerate primers. The 5' primer (5'-GTGAGGCATCAGGTTCAG-3') and 3' primer (5'-DATRTTGTTGGGYTCNCC-3') were designed to cover a portion of mMincle CRD. PCR conditions were 94°C 30s, followed by 35 cycles of 94°C 5s and 50°C 30s. The resulting 311bp PCR product was subcloned into pT7Blue T vector and sequenced. An antisense primer (5'-CCCAGTTCAATGGACAATTCTTG-3') and a sense primer (5'-ACGGCACACCTTTGACAAAGTCTCTG-3') were designed from the sequence and 5'- and 3'- RACE were performed using an adaptor-ligated double stranded cDNA prepared from LPS-stimulated THP-1 cells.

### Example 5 (Northern blot analysis)

Total RNA was separated on 1.0% agarose gels containing 6.0% formaldehyde.

After transfer to a HybondN+ membrane (Amersham Pharmacia Biotech), hybridization was performed at 65°C for 2h in ExpressHyb Hybridization Solution with denatured DNA probe. The membrane was washed twice at 65°C in 2XSSC for 10 min, once at 65°C in 0.2XSSC-0.1%SDS for 10 min, and exposed to BioMax MS film (Kodak, Rochester, NY).

Rsa I-Rsa I cDNA fragment of mMincle (nucleotide 1188-1404), Sph I-Sph I cDNA fragment of mouse NF-IL6 (X62600 nucleotide 135-897), cDNA fragment of mouse MIP-2 (X53798 nucleotide 149-840), cDNA fragment of mouse glyceraldehyde-3-phosphate dehydrogenase (G3PDH; M32599 nucleotide 566-1017) were used as probes. cDNA fragments were radiolabeled with [α-³²P] dCTP (3000 Ci/mmole) by use of the Megaprime DNA labeling system (Amersham Pharmacia Biotech).

### Example 6 (Interspecific Mouse Backcross Mapping)

Interspecific backcross progeny were generated by mating (C57BL/6J x M. spretus) F1 females and C57BL/6J males as described (24). A total of 205 N₂ mice were used to map the Mincle locus.

DNA isolation, restriction enzyme digestion, agarose gel electrophoresis, Southern blot transfer and hybridization were performed essentially as described (25). All blots were prepared with HybondN+ nylon membrane (Amersham Pharmacia Biotech).

The mMincle cDNA fragment (nucleotides 1549-2517) was labeled with [α-³²P] dCTP using a nick translation labeling kit; washing was done to a final stringency of 1.0 XSSCP, 0.1% SDS, 65°C. A fragment of 37 kb was detected in BamHI digested C57BL/6J DNA and a fragment of 6.5 kb was detected in BamHI digested M. spretus DNA. The presence or absence of the 6.5 kb BamHI M. spretus-specific fragment was followed in backcross mice.

A description of the probes and restriction fragment length polymorphisms (RFLPs) for the loci linked to Mincle including Atp6e, Slc2a3, and Cd4 has been reported previously (26, 26. Puech, A., Saint-Jore, B., Funke, B., Gilbert, D. J., Sirotkin, H., Copeland, N. G., Jenkins, N. A., Kucherlapati, R., Morrow, B., and Skoultchi, A. I., Proc. Natl. Acad. Sci. USA 94(26):14608-13,1997).

Recombination distances were calculated using Map Manager, version 2.6.5.

Gene order was determined by minimizing the number of recombination events required to explain the allele distribution patterns.

### Example 7 (Flow Cytometric Analysis)

The mMincle expression vector was constructed in pcDNA3.1(+) (Invitrogen, Carlsbad, CA). To construct pcDNA3. 1(+)-mMincleFlag, forward and reverse primers were devised to introduce an optimal Kozak consensus sequence and Flag epitope, respectively.

The forward primer sequence was 5'-GGTCGACCACCATGAATTCAACCAAATCG-3' and the reverse primer sequence was 5'-CTCACTTGTCATCGTCGTCCTTGTAGTCCAGAGGACTTAT-3'. PCR was performed using double-stranded cDNA generated in the course of RACE as template.

Amplified product was verified by sequencing after subcloned into pT7Blue T vector and excised by Sal I digestion. Then mMincleFlag cDNA fragment was ligated to Xho site of pcDNA3.1(+) with correct orientation. The day before transfection, 293T cells were seeded on 6-well plate at 2.0 X10⁵ cells/well. 4 µg of pcDNA3. 1(+)-mMincleFlag or pcDNA3.1 empty vector was transiently transfected by calcium-phosphate precipitation method. Cells were freed from culture plates using 0.02 % EDTA in PBS at 48 h following transfection and washed in flow cytometry buffer (PBS with 2 % fetal bovine serum and 0.1 % NaN₃). Cells were incubated for 20 min on ice with 15 µg/ml biotin-conjugated anti-Flag M2 antibody (BioM2; Sigma-Aldrich, St. Louis, MO), washed in flow cytometry buffer, and labeled with 5 µg/ml FITC-streptavidin (Pharmingen, San Diego, CA). The cells were harvested 48 h after transfection for flow cytometric analysis. Control consisted of cells treated with FITC-streptavidin alone. After a final wash in flow cytometry buffer, mMincle-Flag expression was analyzed using FACS Calibur using CELLQuest software.

### Example 8 (Western Blot Analysis)

The day before transfection, 293T cells were seeded on 100 mm plate at 2.0 X10⁶ cells. Twenty microgram of pcDNA3. 1 (+)-mMincleFlag or pcDNA3. 1 empty vector was transiently transfected by calcium-phosphate precipitation method. Cells were lysed with lysis buffer containing 0.5% Nonidet P-40, 150 mM NaCl, 1 mM EDTA, and 10 mM Tris-HCl, pH 7.4. Cell lysates were immunoprecipitated with protein-G Sepharose (AmershamPharmacia Biotech) together with 10 µg/ml anti-Flag M2 antibody. Immunoprecipitates were washed four times with lysis buffer and suspended with Laemmli sample buffer. After boiling for 5 min, samples were separated on a gradient (10-20%) SDS-polyacrylamide gel, and electrically transferred to a nitrocellulose membrane. The membrane was incubated with BioM2 antibody, subsequently treated with horseradish peroxidase-conjugated streptavidin (Genzyme), and then analyzed for immunoreactivity by the enhanced chemiluminescence detection system (Dupont, Boston, MA).

### Example 9 (Isolation of mMincle 5 '-flanking region and reporter gene assays)

A 129/Sv mouse liver genomic DNA library in the λ Fix II vector was purchased from Stratagene. Approximately 1 X 10⁶ plaques were screened with the ³²P-labeled Eco RI-Eco RI cDNA fragment of mMincle (nucleotides 126-496). Positive plaques from the genomic library were enriched after two further rounds of screening. DNA from two independent clones was purified using the Wizard prep kit (Promega). Digestion with several restriction enzymes revealed that these two clones contain the identical sequence. Following digestion with Bam HI and Sal I, five resulting fragments (5.4kb, 5.1kb, 3.2kb, 0.8kb and 0.6kb) were subcloned into pBluescript KS (+) and sequenced.

The 5.1kb Bam HI-Bam HI fragment containing exon I and 5'-flanking region was designated pBS/Baml-8 and employed for promoter-luciferase construction. Appropriate 5'-primers and common 3'-primer were synthesized to amplify the promoter regions of mMincle. The following primers were used to generate pGL3-1783/+69, pGL3-1190/-69, pGL3-240/+69, and pGL3-61/+69; -1783 (5'-CGACGCGTGGTTTGCAGCCCCATAGGAG-3'), -1190(5'-CGACGCGTATGATGGCACACCATGATAG-3'), -240 (5'-CGACGCGTAAATCGGGACCAAG3ITAGAC-3'), -61(5'-CGACGCGTCAAGAGAGGAAATTCTGAC-3') and

-69(5'-GAAGATCTCCCCTGGAAAGTGAGTCTTG-3').

Amplified products were subcloned into pT7Blue T vector and sequenced for confirmation. The inserted fragments were cut out with Mlu I and Bgl II digestion and ligated to pGL3 basic vector (Promega, Madison, WI) at the same restriction sites. To create pGL3-166/+69, pGL3-240/+69 was digested with Mlu I and Aat I. blunt-ended and re-ligated. NF-IL6 binding site mutation was generated by Quick Change Site Directed Mutagenesis Kit (Stratagene). The mutagenic primer (with altered nucleotides underlined) was 5'-CCTTGTCCTTGTGCCCCAGAGGAAATTCTG-3'. The mutated construct was confirmed by sequencing.

Transient transfection into NFIL6M1 cells and luciferease assay were done as described previously (21, 21. Matsumoto, M., Sakao, Y., and Akira, S., Int. Immunol., 10(12):1825-35,1998).

### Example 10 (Primer extension analysis)

Primer extension was performed as described (27, 27. Sambrook, J., Fritsch, E. F., and Maniatis, T., (1989) Molecular Cloning: A laboratory Manual, Second Ed., Cold Spring Harbor Laboratory Press, Cold Spring Harbor, NY). Briefly, an oligonucleotide primer complementary to nucleotides 81-112 of Mincle cDNA was synthesized, and end-labeled with [γ-³²P]ATP and T4 polynucleotide kinase. Ten microgram of total RNA from LPS-stimulated PMΦ was hybridized to 10⁴ cpm of the labeled oligonucleotides in 10 mM Pipes. pH6.4. 1mM EDTA pH 8.0, and 0.4 M NaCl at 30°C for 16 h. Following ethanol precipitation, the samples were dissolved in 20 µl reaction buffer containing 50 mM Tris/HCl pH 8.3, 10 mM MgCl₂, 1 mM dithiothreitol, 75 mM KCl, 1 mM dNTPs and 20 U RNase inhibitor.

Reverse transcription was performed at 42°C for 30 min by adding 200 U Superscript II (Gibco/BRL, Gaitherburg, MD). The extension products were ethanol precipitated and analyzed on 6% polyacrylamide 7 M urea sequencing gels.

A sequence reaction was set up separately with the same non-radiolabeled primer, using the template of genomic clone pBS/Bam1-8, which covered the mMincle 5'-flanking region, and was run in parallel with the extension preducts on the same sequencing gel.

### Example 11 (Electrophoretic Mobility Shift Assays)

The following single-stranded oligonucleotides were synthesized for electrophoretic mobility shift assays:
P1 (position -76 to -45 of the mMincle promoter), 5'-CCTTGTCCTTGTGCAAGAGAGGAAATTCTG-3' and 5'-GTCAGAATTTCCTCTCTTGCACAAGGACAAGG-3'; mP1, 5'-CCTTGTCCTTGTGCCCCAGAGGAAATTCTG-3' and 5'-GTCACGAATTTCCTCTGGGGCACAAGGACAAGG-3'; IL6 (position -165 to -138 of the human IL-6 promoter), 5'-GGACGTCACATTGCACAATCTTAATAAT-3' and 5'-ATTATTAAGATTGTGCAATGTGACGTCC-3'. Underlined nucleotides represent the mutant sequences. Complementary DNA oligonucleotides were annealed by heating in a buffer containing 20mM Tris-HC1, pH 7.5, 10mM MgCl₂, 50mM NaCl at 75°C for 5 min and cooling at room temperature.

Probes were then labeled by filling in with [α-³²P] dCTP using Klenow fragment. NFIL6 Ml nuclear extracts were prepared as described previously (21, 21. Matsumoto, M., Sakao, Y., and Akira, S., Int. Immunol., 10(12):1825-35, 1998). pGEX-NFIL6 plasmid inserting human NF-IL6 ((7), amino acids 24-345) downstream of the glutathione S-transferase (GST) gene in pGEX-4T-2 is a generous gift from S. Hashimoto. An empty pGEX- 4T-2 and pGEX-NF-IL6 plasmids were introduced into E. coli BL21 to produce GST and GST-NF-IL6 protein, respectively. pGEX-transformed E. coli were grown to mid-exponential phase, induced with 1 mM IPTG, and lysed by sonication in hypotonic buffer. The GST-fusion proteins were purified from the bacterial lysate by incubation with glutathione-coupled Sepharose beads (AmershamPharmacia Biotech). Protein concentration was determined by BCA™ Protein Assay Reagent (Pierce, Rockford, IL). Nuclear extracts or GST-fusion proteins were incubated with 1X 10⁴ cpm of the labeled DNA probe in 25 µl of binding buffer containing 10 mM Hepes-KOH, pH 7.8, 50 mM KCl, 1mM EDTA, pH8.0, 5mM MgCl₂, 10% Glycerol and 3µg of poly (dI-dC) (AmershamPharmacia Biotech). Competition assays were carried out in the same manner, except that the above reaction mixture was preincubated with a 100-fold molar excess of unlabeled competitor oligonucleotides for 30 min at 4°C before the addition of the labeled probe. Supershift assays were performed using 200 ng of anti-C/EBPβ antibody (C-19; Santa Cruz Biotechnology, Santa Cruz, CA) and preincubated with the above reaction mixture at 4°C for 30 min prior to the addition of the labeled probe. Samples were loaded on native 5 % polyacrylamide gels, and electrophoresis was carried out at 30 mA in 25 mM Tris, pH 8.5, 190 mM glycine, and 1 mM EDTA. Gels were subsequently dried for autoradiography.

### Industrial Applicability

The present invention defines a target product downstream of nuclear factor. NF-IL6, and provides the target product as a novel polypeptide belonging to C-type lectins, i.e., Mincle (macrophage inducible C-type lectin). And the present invention have studied various natures of the novel polypeptide, "Mincle" and demonstrated that Mincle has an action to activate macrophages. Therefore, the present invention provides the novel protein having an activation action of macrophages and useful for the treatment and prevention of various immune disorders, inflammatory diseases and the like.

Also the present invention provides a novel gene encoding "Mincle" protein of the present invention. Further, the present invention provides medicinal compositions containing "Mincle".

The present invention provides an antibody against "Mincle" of the present invention.

## Claims

1. A protein having an amino acid sequence shown in the sequence No. 2 in the sequence table, or an amino acid sequence wherein one or two or more amino acids are added, substituted or deleted in the amino acid sequence, and having an activation ability of macrophages.

2. The protein according to claim 1 inducible by nuclear factor NF-IL6.

3. A gene encoding the protein according to claim 1 or 2.

4. The DNA according to claim 3 having a base sequence shown in the sequence No. 1 in the sequence table.

5. The DNA capable of hybridizing to the DNA according to claim 3 or 4 under a stringent condition.

6. A polynucleotide consisting of at least 14 bases of the DNA according to claim 3 or 4.

7. The labeled polynucleotide according to claim 6.

8. A medicinal composition containing the protein according to claim 1 or 2 and pharmaceutically acceptable carriers.

9. The medicinal composition according to claim8 for the treatment of the disorders capable of being treated or prevented by an activation action of macrophages.


SEQUENCE LISTING

<110> Japan Science and Technology Corporation

<120> NOVEL C-TYPE LECTIN AND GENE THEREOF

<130> JA904071

<150> JP 11-293724
<151> 1999-10-15

<160> 2

<210> 1
<211> 2517
<212> DNA
<213> Mouse

<400> 1
cggtctgtta ctcttgaact tttaaaaaga gggccaagga ttcaccattc aagactcact 60

ttccaggggc tctttctaaa ctgagaagag aaggaaaagg aagaaaggca ggaaaaagga 120

agaatgaatt caaccaaatc gcctgcatcc caccacacag agagaggatg cttcaaaaac 180

tcccaagtgc tctcctggac gatagccggg gcctccatcc tgtttctcag tggctgtttc 240

atcaccagat gtgtcgtaac atatcgcagc tctcaaattt ccgggcagaa cttacagcca 300

catagaaata ttaaggagct ttcctgctac agtgaggcat caggttcagt caagaattgc 360

tgtcctttga actggaaaca ttatcaatct agttgttatt ttttctctac gacaaccttg 420

acctggtcat caagtttaaa gaattgctca gacatggggg ctcacctggt ggttatcgac 480

acacaggaag agcaggaatt cctttttcgc acaaaatcta aaggaaaaga gttttatatt 540

ggactgacag accaggtggt ggagggtcag tggcaatggg aggatgatac acctttcaca 620

gagtccctga ccttctggga tgctggggag cccaacaata tagttttggt ggaggactgt 680

gccaccataa gggactcttc aaactccagg aagaactgga atgataatcc ctgtttctac 720

agtatgcctt ggatttgtga gatgccagaa ataagtcctc aggactaagt gcaaggaaat 780

acaagggaca tggcttacat gcatgaagaa gaacaagagt gaatgtaata acaaccaaaa 840

tccaacataa gaaaatatct atcaggcatc agaaggactg cacatgtatg tattactggg 900

acataagtaa aaagacttgt tcccattgct aaaagtccac agcattgtct gatggtcttg 960

ccataacctg aaagatctct ttttagactg tacagatcaa ttctctaaca aatgcaacaa 1020

gaagaaaggg attctccttt tcacatctgt cttgcacatc tgtcttgctc atgagaattg 1080

atatgaagga agaggtagaa agcagatgtc tgtataaaga gactttaatg gtcactatgt 1140

catcctgttc tttctacatc cttggctcta gcttatctat ctatcagtac atagatcact 1200

tctgtgttct ccaacagtga ggaagatgca tctttgagtc tttaaactta cctgccgctt 1260

gggagaatgg catggccttc agcaaggaca tctccatatg gaaaggccgg tcaaacttca 1320

gttcctaaca gattgtgatc tagtccactc ttcctggagc cccatttct ctgtgttctc 1380

ttctataaac tggatttcac ctgtacttgt atctactgcg caagtagaac ctgctcagta 1440

ggttcaaagt gaaattattt aaaaattcat gttcacattt ttctgtctca ggactgcatt 1500

tattgcatga tattctgtca atatagacca tgtttcttcc agacaaagcc cattaggaac 1560

ttcagcagca gtcacacatt gtaataaaca tgtatccttg agtaggaaaa ttaaactaaa 1620

taaattaatt tgtcatatta gcactcatta cgagcacttc tattagactt tctcacaatc 1680

tgattttgaa attgataacc ttattttaaa tacaaatata tcttacaacc acacatttga 1740

cttctctttt taaaattatt tttgtttgaa aatttgtgc atttatataa ttcactttta 1800

atgaacccat ccttactctc ctcactacaa cctgctccta tccacctcac tgattttccc 1860

tcccaatttc atgtgctcct ctttgtttta aacccactct atctgctcag tgcttcctga 1920

atgcacttga gtataaggct ttctactgga ccatagcctc tcggcaacca catcccatac 1980

tccacctgct ccagcaggga acaatagcca attgaccatc ttcagctgag gatggatttc 2040

atgagctcca tgccattcat gctggaattt gggttgtttt atgtaacctt tattatattg 2100

tgctatctct tctgtatccc tagaatctct aggagcttca tattaaaaga ttctgaattc 2160

catcaaaggg cacacaaaga aatcacaaag accatgtggt ttctgtcctt gggtttattt 2220

gcaaggttca ttacactcct tgacttgtat atattgtgac atccctccat ctctaggatg 2280

aaactgaagt gatcatgata gataactttt ggatcttttc acttttctat tgctgtgatg 2340

aaacatgacc caaaactatc ttgggccggg aaaattttaa tttatcttaa cataaatttc 2400

ctatttagaa attggacagg tggggaatca tccaattgaa actcgaaaaa tgtagtgttt 2460

tgtacattag gttacagaaa aacaacttta gccacaaaaa taaagtaata aactatt 2517

<210> 2
<211> 214
<212> PRT
<213> Mouse

<400> 2
Met Asn Ser Thr Lys Ser Pro Ala Ser His His Thr Glu Arg Gly Cys
1               5                   10                  15
Phe Lys Asn Ser Gln Val Leu Ser Trp Thr Ile Ala Gly Ala Ser Ile
            20                  25                  30

Leu Phe Leu Ser Gly Cys Phe Ile Thr Arg Cys Val Val Thr Tyr Arg
        35                  40                  45
Ser Ser Gln Ile Ser Gly Gln Asn Leu Gln Pro His Arg Asn Ile Lys
    50                  55                  60
Glu Leu Ser Cys Tyr Ser Glu Ala Ser Gly Ser Val Lys Asn Cys Cys
65                  70                  75                  80
Pro Leu Asn Trp Lys His Tyr Gln Ser Ser Cys Tyr Phe Phe Ser Thr
                85                  90                  95
Thr Thr Leu Thr Trp Ser Ser Ser Leu Lys Asn Cys Ser Asp Met Gly
            100                 105                 110
Ala His Leu Val Val Ile Asp Thr Gln Glu Glu Gln Glu Phe Leu Phe
        115                 120                 125
Arg Thr Lys Pro Lys Arg Lys Glu Phe Tyr Ile Gly Leu Thr Asp Gln
    130                 135                 140
Val Val Glu Gly Gln Trp Gln Trp Val Asp Asp Thr Pro Phe Thr Glu
145                 150                 155                 160
Ser Leu Ser Phe Trp Asp Ala Gly Glu Pro Asn Asn Ile Val Leu Val
                165                 170                 175
Glu Asp Cys Ala Thr Ile Arg Asp Ser Ser Asn Ser Arg Lys Asn Trp
            180                 185                 190
Asn Asp Ile Pro Cys Phe Tyr Ser Met Pro Trp Ile Cys Glu Met Pro
        195                 200                 205
Glu Ile Ser Pro Leu Asp
    210